# EUROPEAN PATENT APPLICATION

(11) **EP 3 360 957 A1**
(43) Date of publication of application: **15.08.2018**
(21) Application number: 15905664.7
(22) Date of filing: 08.10.2015
(51) Int. Cl.: C12N 5/0775, C12N 5/00

(54) **COMPOSITION FOR RAPIDLY SEPARATING ADIPOSE TISSUE-DERIVED STROMAL CELLS**

(71) Applicant: Kaohsiung Medical University, Kaohsiung 807 (TW)
(72) Inventor: WANG, Yao-Hsien, Kaohsiung City 825 (TW); CHEN, Chung-Hwan, Kaohsiung City 801 (TW); HO, Mei-Ling, Kaohsiung City 813 (TW); CHANG, Je-Ken, Kaohsiung City 807 (TW)
(74) Representative: Lang, Christian
(86) International application number: PCT/CN2015/091449
(87) International publication number: WO 2017/059566

(57) **Abstract**

The present invention provides a composition for isolating adipose-derived stromal cells, which comprises a Type I collagenase of 0.5-8% (v/v); a Trypsin of 0.1-0.6% (v/v); and a metal ion chelating agent of 0.01-0.2% (v/v). The present invention further provides a method for isolating adipose-derived stromal cells, which method comprises obtaining an adipose tissue; treating the adipose tissue with the composition of the present invention; centrifuging the adipose tissue; and isolating the adipose tissue to obtain the adipose-derived stromal cells. The present invention facilitates rapid isolation of mesenchymal stromal cells within a short period of time in operating rooms and can be applied to regenerative medicine in the future.

## Description

### CROSS-REFERENCE TO RELATED APPLICATIONS

This patent application is a U.S. National Stage Application of PCT/CN2015/091449 filed October 08, 2015, the content of which is incorporated herein by reference in it entirety.

### FIELD OF THE INVENTION

The present invention relates to a composition for isolating adipose-derived stromal cells and a method for rapidly isolating adipose-derived stromal cells

### BACKGROUND OF THE INVENTION

Adipose-derived stem cells (ADSCs), which are adult stem cells widely used in tissue engineering and regenerative medicine, have the potential of multi-directional differentiation which shares similarities to those of bone marrow mesenchymal stem cells. At present, the method commonly used in laboratories for isolating and cultivating ADSCs is to isolate and derive the cells from adipose tissues. This method has been used for several decades. Generally, a tissue is removed, cut and combined with collagenase to digest the extracellular matrix in order to release single cells from the tissue. It takes about 12 hours or longer (usually overnight) to decompose the tissue. In addition to time consumption, survival of cells is compromised when the cells stay in an environment with enzymes for a long period of time, which often results in unstable physiological biology condition or even death of the cells, reducing the total number of cells that can be harvested. Mature cells such as red blood cells are removed by mechanical and enzymatic treatments and then cultivated in a medium containing 10% fetal bovine serum. The disadvantages of this stem cell culture method is that the method is complex, the number of extracted stem cells is small, the purity is not high, and the proliferation of subcultured cells is slow.

Non-adipose single cells derived from liquid adipose tissue obtained by liposuction technique, treated with collagenase for digestion, and followed by centrifugation are referred as stromal vascular cells (SVF). These single cells can be obtained rapidly, however, the ultrasonic oscillating method, used for liposuction may cause great damage to the cells and affect cell survival rate.

Recently, regenerative medicine, orthopedic research in particular, emphasizes that mesenchymal stem cells can be used as a cell source for bone regeneration and repair. Therefore, transplantation of autologous stem cells, particularly transplantation of autologous adult adipose-derived stem cells, is the future trend of stem cell transplantation. However, the procedure required for isolating and selecting mesenchymal stem cells from autologous tissues is rather complicated and time consuming. To amplify sufficient number of autologous mesenchymal stem cells *in vitro* for use substantially requires good laboratory practice (GLP) in the laboratory site, the time consumption required for *in vitro* operation is long and bacterial or viral infection rate needs to be restricted. The foregoing factors limit the clinical applications of stem cells. The present invention will facilitate rapid isolation of mesenchymal stromal cells in operating rooms within a short period of time and can be applied to regenerative medicine.

### DETAIL DESCRIPTION OF THE INVENTION

Unless otherwise defined in the present context, the scientific and technical terms used in the present invention should possess the meaning commonly known by any one of ordinary skill in the art. The meaning and scope of the terms should be clear; nevertheless, in any circumstance of discrepancy in the meaning, definition provided in the present context precedes those defined in any other dictionaries or external reference

As used herein, the term "a plurality of' is used to describe the number of components or units of the present invention. Unless expressly stated otherwise, the term should be read as two or more.

As used herein, the terms "a" or "an" are used to describe the components and constituents of the present invention. This term is used only for the convenience of description and to provide the basic concepts of the present invention. This description should be read to include one or at least one, and unless expressly stated otherwise, singular also includes the plural.

As used herein, the term "or" means "and/or."

In order to overcome the deficiencies of prior arts, the present invention provides a composition and a method which alleviate patients' pain, fulfill clinically safe application requirements, and rapidly isolate mesenchymal stromal cells within a short period of time.

The present invention provides a composition for isolating adipose-derived stromal cells, which comprises a Type I collagenase, the concentration is 0.5-8% (v/v); a Trypsin, the concentration is 0.1-0.6% (v/v); and a metal ion chelating agent, the concentration is 0.01-0.2% (v/v).

The term "metal ion chelating agent" as used herein is selected from ethylenediaminetetraacetic acid (EDTA) or sodium salts thereof, ethylene glycol-bisaminoethyl ether tetraacetic acid (EGTA) or sodium salts thereof, diethyltriaminepentaacetic acid (DTPA) or sodium salts thereof, polyphosphates, organic phosphates, phosphates, polyacrylates, organic phosphates, sodium gluconate, or a combination thereof.

In one preferred embodiment, the composition for isolating adipose-derived stromal cells of the present invention, wherein the metal ion chelating agent is EDTA.

In another preferred embodiment, the composition for isolating adipose-derived stromal cells of the present invention, which comprises 2-4% (v/v) of Type I collagenase, 0.1-0.3% (v/v) of Trypsin and 0.01-0.1% (v/v) of EDTA.

In one embodiment, the composition for isolating adipose-derived stromal cells of the present invention is sterile.

In one embodiment, the composition for isolating adipose-derived stromal cells of the present invention has an effect of sufficiently releasing the adipose-derived stromal cells while protecting these cells from being damaged.

The present invention further provides a method for isolating adipose-derived stromal cells, which comprises the steps of: (a) obtaining an adipose tissue; (b) adding the composition for isolating adipose-derived stromal cells of the present invention, homogenizing and allowing for reaction to obtain a digested tissue mixture, wherein the composition comprises a Type I collagenase which is 0.5-8% (v/v); a Trypsin which is 0.1-0.6% (v/v); and a metal ion chelating agent which is 0.01-0.2% (v/v); (c) centrifuging the digested tissue mixture of step (b), removing impurities to obtain a filtrate containing the adipose-derived stromal cells; (d) adding a hypotonic solution to the filtrate of step (c) to obtain a hemocyte-free adipose-derived stromal cells filtrate; and neutralizing the filtrate of step (d) and centrifuging to obtain the adipose-derived stromal cells.

In one embodiment of the present invention, the adipose tissue is obtained from an individual, wherein the individual comprises a human or an animal.

In one preferred embodiment of the present invention, the metal ion chelating agent is EDTA.

In another preferred embodiment of the present invention, the concentration of Type I collagenase is 2-4% (v/v), the concentration of Trypsin is 0.1-0.3% (v/v), and the concentration of EDTA is 0.01-0.1% (v/v).

In one embodiment of the present invention, the method for isolating the adipose-derived stromal cells is sterilized.

In one preferred embodiment, the method for isolating the adipose-derived stromal cells is further characterized in that the total reaction time for sufficiently digesting the adipose tissue is one hour or less.

In another preferred embodiment, the method for isolating the adipose-derived stromal cells of the present invention is capable of isolating one million adipose-derived stromal cells from 5 grams of adipose tissue.

In one embodiment, the method for isolating the adipose-derived stromal cells of the present invention, wherein the adipose-derived stromal cells are capable of differentiating into adipose cells, hematopoietic cells, vascular endothelial cells, osteoblasts, chondroblasts, nerve cells or epithelial cells.

In one preferred embodiment, the method for isolating the adipose-derived stromal cells of the present invention, wherein the adipose-derived stromal cells are capable of differentiating into adipose cells, osteoblasts or chondroblasts.

In one preferred embodiment, the method for isolating the adipose-derived stromal cells of the present invention has an effect of sufficiently releasing the adipose-derived stromal cells while protecting these cells from being damaged.

### DETAIL DESCRIPTION OF THE DRAWINGS

Figure 1 shows a schematic flow chart of one embodiment of the method for isolating adipose-derived stromal cells of the present invention.
Figure 2 shows the results of the number of mesenchymal stromal cells isolated from 5 grams of subcutaneous adipose tissue extracted from the inner thighs of SD rats by different concentrations of enzyme formulations for one hour.
Figure 3 shows the results of the number of mesenchymal stromal cells isolated from 5 grams of subcutaneous adipose tissue extracted from the inner thighs of guinea pigs by different concentrations of enzyme formulations for one hour.
Figure 4 shows the differentiation capability analysis of the adipose-derived stem cells selected from the selected adipose-derived stromal cells.
Figure 5 shows the expression level of biomarkers on the cell surface of the isolated adipose-derived stem cells.
Figure 6 shows the effect of different concentrations of enzyme formulations on the survival of mesenchymal stromal cells.
Figure 7 is a comparison when the content of the composition of the present invention is replaced with different species of enzymes.

### DESCRIPTION OF THE CODINGS IN THE DRAWINGS

A 0.5% Type I collagenase + 0.05% Trypsin + 0.02% EDTA
B 0.5% Type I collagenase + 0.1% Trypsin + 0.02% EDTA
C 0.5% Type I collagenase + 0.2% Trypsin + 0.02% EDTA
D 1% Type I collagenase + 0.1% Trypsin + 0.02% EDTA
E 2% Type I collagenase + 0.1% Trypsin + 0.02% EDTA
F 2.5% Type I collagenase + 0.1% Trypsin + 0.02% EDTA
G 3% Type I collagenase + 0.1% Trypsin + 0.02% EDTA
H 4% Type I collagenase + 0.1% Trypsin + 0.02% EDTA
E' 2% Type IV collagenase + 0.1% Trypsin + 0.02% EDTA
F' 2.5% Type IV collagenase + 0.1% Trypsin + 0.02% EDTA
G' 3% Type IV collagenase + 0.1% Trypsin + 0.02% EDTA
I' 3.5% Type IV collagenase + 0.1% Trypsin + 0.02% EDTA
H' 4% Type IV collagenase + 0.1% Trypsin + 0.02% EDTA

### EXAMPLES

The present invention may be implemented in many different forms and should not be construed as limited to the examples set forth herein. The examples below are non-limiting and are merely representative of various aspects and features of the present invention.

The One-way Analysis of Variance (ANOVA) was used to test for statistical differences and the Scheffe's test was used for multiple comparisons. Significant difference is defined as a significant probability value (p-value) <0.05.

### Example 1 Rapid isolation of mesenchymal stromal cells

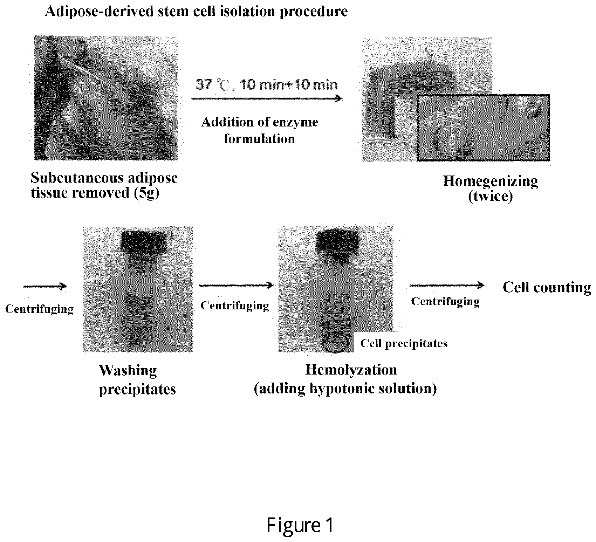

The schematic flow chart as shown in Figure 1 shows the rapid isolation of mesenchymal stromal cells from adipose tissues of Sprague-Dawley rats (SD rats) and Genia pigs to which the enzyme formulations of the present invention were applied.

5 grams of adipose tissue were removed from the inner thighs of SD rats and guinea pigs, an equal amount (about 5 mL) of enzyme formulation buffer solution of the present invention (0.5-3% (v/v) of type I collagenase, 0.1-0.3% (v/v) of trypsin and 0.02% (v/v) of EDTA dissolved in a phosphate buffer solution (PBS)) was added; placed in a sterile tissue homogenizer (GentleMACs, Miltenyi Biotec, Bergisch Gladbach, Germany) to homogenize the tissues into single cells for 1 minute and then soaked in a 37 water bath to react for 10 minutes, then placed in a sterile tissue homogenizer again for 1 minute and soaked in a 37 water bath for 10 minutes. Subsequently, after it was centrifuged at (300 × g) for 10 minutes, the upper layer containing lipid, connective tissues, or non-homogenized tissues were removed, washed with PBS (5 mL) and then centrifuged (300 × g) for 10 minutes, an equal amount of hypotonic solution (5 mL) was added, stood at room temperature for 5 minutes to remove blood cells and then 10mL of PBS buffer solution was added to neutralize the reaction. The solution was removed after it was centrifuged at 3,000 rpm for 10 minutes, the cells in the lower layer were re-suspended in 1 mL of PBS and then sampled to count the number of cells. The total reaction time was less than one hour.

### Example 2 Mesenchymal stromal cells yield analysis

20 µL was removed from the adipose-derived stromal cells sample isolated in Example 1, 20 µL of Trypan blue was added, mixed thoroughly for staining, dripped into a cell counting dish to count viable cells (non-stained cells).

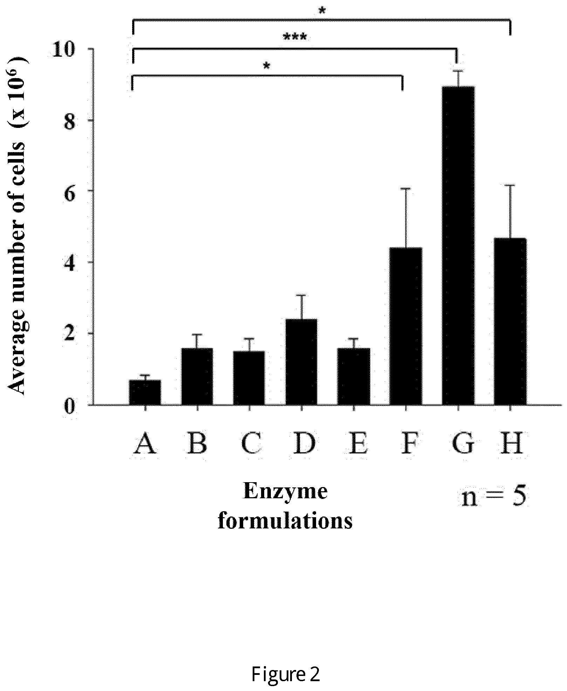

Fig. 2 shows the number of mesenchymal stromal cells isolated by different concentrations of enzyme formulations for one hour from 5 grams of subcutaneous adipose tissues removed from the inner thighs of SD rats. Results showed that when the enzyme formulation containing 3% (v/v) of Type I collagenase, 0.1% (v/v) of Trypsin and 0.02% (v/v) of EDTA (encoded as G) was used for one hour, the number of extracted cells was the highest, approximately 9×10⁶ cells/mL could be isolated.

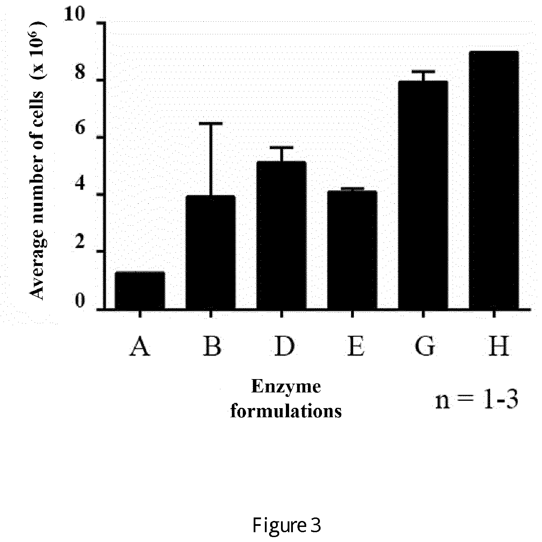

Fig. 3 shows the number of mesenchymal stromal cells isolated by using different concentrations of enzyme formulations for one hour from 5 grams of subcutaneous adipose tissues removed from the inner thighs of guinea pigs. Results showed that when the enzyme formulation containing 3-4% (v/v) of type I collagenase, 0.1% (v/v) of Trypsin and 0.02% (v/v) of EDTA (encoded as G, H) was used for one hour, the number of extracted cells was the highest, approximately 8×10⁶ to 8.5×10⁶ cells/mL could be isolated.

### Example 3 Differentiation capability of adipose-derived stem cells after isolation

According to the definition of mesenchymal stem cell published by the International Association of Mesenchymal Stem Cells in 2006, a mesenchymal stem cell has three characteristics:
1. the cell must be attached to a cell culture dish;
2. surface antigens are required to express CD105, CD73, or CD90 but not CD45, CD34, CD14, or CD11b, CD79a, or CD19, or HLA-DR;
3. after being induced, the mesenchymal stem cell is required to be capable of differentiating into adipocytes, osteoblasts and chondrocytes.

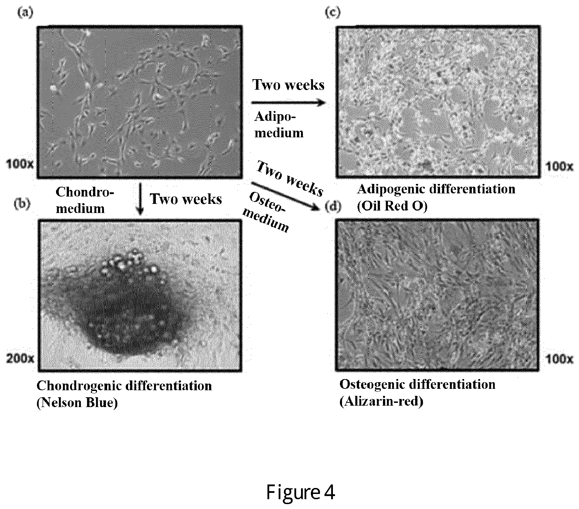

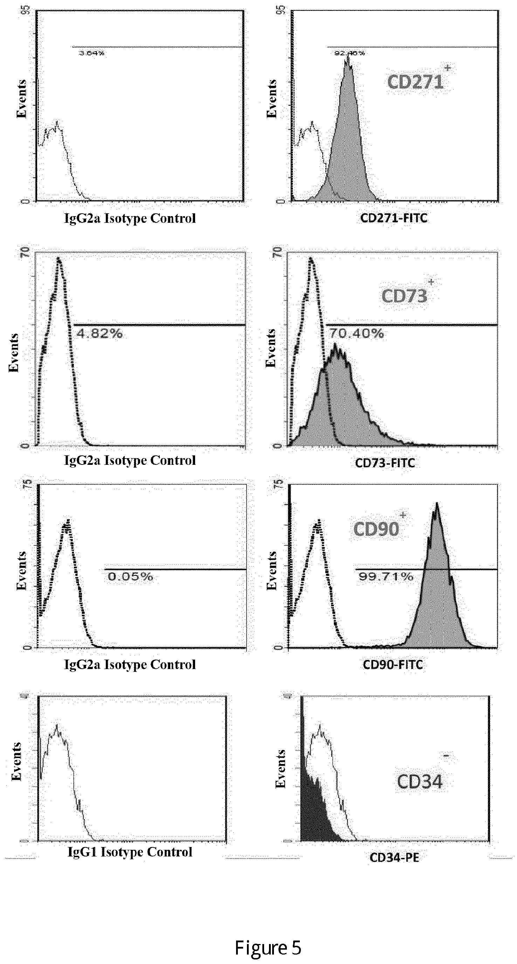

When the adipose-derived stromal cells isolated in Example 1 were cultivated in a selective medium (Kerationcytr-SFM; Product Number: 10724-001; GIBCO, New York, USA) (mesenchymal stem cells were selectively retained, non-mesenchymal stem cells underwent apoptosis), adipose-derived mesenchymal stem cells could be isolated after one week of cultivation. As shown in Figure 4(a), the isolated adipose-derived mesenchymal stem cells were adherent cells. When the isolated adipose-derived stromal cells were analyzed by using a flow cytometry, the expression levels of CD271, CD73 and CD90 on the cell surface were significantly higher than that of the control group, and the expression level of CD34 was lower than that of the control group, as shown in Figure 5. Therefore, the isolated adipose-derived stromal cells belong to mesenchymal stem cells. The following further examined whether the mesenchymal stem cells were capable of being induced to differentiate into adipocytes, osteoblasts and chondrocytes.

After being subcultured, the isolated adipose-derived mesenchymal stem cells were cultured for two weeks in different differentiation-inducing culture media (Adipo-medium: a medium inducing differentiation into adipocytes; Osteo-medium: a medium inducing differentiation into osteoblasts; and Chondro-medium: a medium inducing differentiation into chondrocytes; wherein Adipo-medium: DMEM medium (Dulbecco's Modified Eagle Medium), 10% of fetal bovine serum (FBS), 1% of penicillin/streptomycin, 500 µM of 3-isobutyl-1-methylxanthine IBMX, 1 µM of dexamethasone, 1 µM of indomethasin, 10 Mg/mL of insulin; Chondro-medium: DMEM medium (Dulbecco's Modified Eagle Medium), 10% of fetal bovine serum (FBS), 1% of penicillin/streptomycin, 50 nM of L-ascorbic acid-2-phosphate (L-Ascobate-2-phosphate), 6.25 µg/mL of insulin, 10 ng/mL of TGF-β; and Osteo-medium: DMEM medium (Dulbecco's Modified Eagle Medium), 10% of fetal bovine serum (FBS), 1% of penicillin Streptomyces , 50 µM of L-ascorbyl-2-phosphate , 0.1 µM of dexamethasone, 10 mM of β-glycerophosphate (β-glycerophosphate).

The differentiation capability of mesenchymal stem cells into chondrocytes was evaluated by using a glycosaminoglycan (abbreviated as GAG) assay, the GAG test was carried out with Alcian blue staining. After two weeks of cultivation in Chondro-medium, the old medium was removed and fixed with 10% of formalin or 4% of paraformaldehyde for 10 minutes. It is washed twice with distilled water (3 mL), and then shaken with 3% of acetic acid (3 mL) for 5 minutes. After all liquids were removed, Nelson Blue (1%) was added into the dish, shaken for 15 minutes, after the stain was removed and then washed with distilled water (3 mL) for 2-3 times, photos were taken.

As shown in Figure 4(b), the extracellular matrix GAG was stained by Nelson blue, the stained GAG was blue and the cells aggregated, which were characteristics of chondrocytes.

The ability of the mesenchymal stem cells to differentiate into adipocytes was evaluated by staining oil droplets in the adipocytes with Oil Red O. Two weeks after the Adipo-medium incubation, the old medium was removed and fixed in 10% formalin or 4% paraformaldehyde for 10 minutes. Distilled water (3 mL) was used to wash for 2 to 3 times. After all the liquids were removed, Oil Red O (0.5%) was added into the dish, shaken for 10 minutes, distilled water (3 mL) was used to wash 2-3 times to remove stains. Photos were taken for record after distilled water was added.

As shown in Figure 4(c), the oil droplets in the stained cells were in the form of red dots, which is a characteristic of the adipocytes.

Alizarin-red staining was used to examine the effect of isolated adipose-derived mesenchymal stem cells on osteogenesis. After two weeks of incubation in Osteo-medium, the old medium was removed and fixed in 4% formalin for 15 minutes. Distilled water (3 mL) was used to wash for two times. After all the liquids were removed, 2% bismuth red solution was added into the dish, reacted at room temperature for 20 minutes, photos were taken after distilled water was used to wash for 2-3 time to remove stains.

As shown in Figure 4(d), the calcium deposition generated by the stained cells was in the form of red dots, which is a characteristic of osteoblasts.

### Example 4 Effect of different concentrations of enzyme formulations on the survival of mesenchymal stromal cells

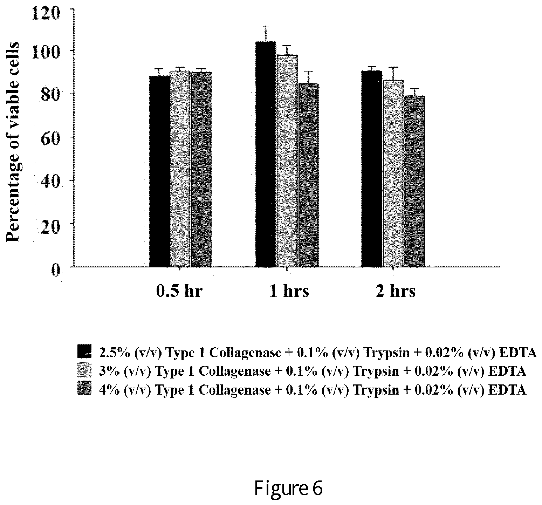

When the adipose-derived stromal cells isolated in Example 1 were cultivated in a selective medium (Kerationcytr-SFM; Product Number: 10724-001; GIBCO) (mesenchymal stem cells were selectively retained, non-mesenchymal stem cells underwent apoptosis), adipose-derived mesenchymal stem cells could be isolated after one week of cultivation. The following three preferred enzyme formulations which yielded more adipose-derived stromal cells in Example 1 were used to carry out citytoxicity tests:
1. enzyme formulation of 2.5% (v/v) Type I collagenase, 0.1% (v/v) Trypsin, and 0.02% (v/v) EDTA;
2. enzyme formulation of 2. 3% (v/v) Type I collagenase, 0.1% (v/v) Trypsin and 0.02% (v/v) EDTA; and
3. enzyme formulation of 4% (v/v) Type I collagenase, 0.1% (v/v) Trypsin and 0.02% (v/v) EDTA.
10⁵ adipose-derived mesenchymal stem cells were placed in these enzyme formulations and incubated for 0.5, 1 and 2 hours. After 0.5, 1 and 2 hours of cultivation, cells were removed and subjected to Trypan-blue staining for calculating cell survival rate. As shown in Figure 6 , the formulations did not show significant cytotoxicity.

### Example 5 Comparison of Substitution of Different Enzymes

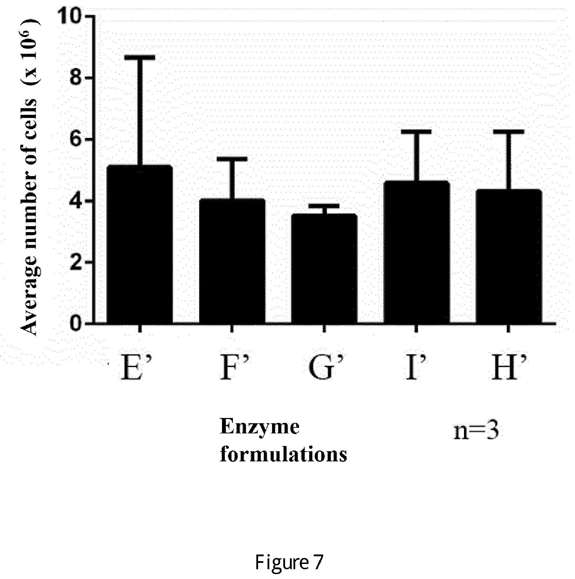

The enzymes in the enzyme formulations were replaced with similar but different species of enzyme while the concentrations remained the same: 2% (v/v) of Type 4 collagenase, 0.1% (v/v) of Trypsin, 0.02% (v/v) of EDTA ; 2.5% of Type 4 collagenase, 0.1% of Trypsin, 0.02% of EDTA; 3% of Type 4 collagenase, 0.1% of Trypsin, 0.02% EDTA; 3.5% Type 4 of collagenase, 0.1% of Trypsin, 0.02% of EDTA; 4 % of Type 4 collagenase, 0.1% of Trypsin, 0.02% of EDTA, and the adipose-derived stromal cells isolation method disclosed in Example 1 was used in this example. The results were shown in Figure 7, the number of cells yielded by the formulation of the this example was unable to match the number of cells yielded by the formulation of 3% (v/v) Type 1 collagenase, 0.1% (v/v) Trypsin, and 0.02% (v/v) EDTA. Therefore, enzyme formulation of 3% (v/v) Type I collagenase, 0.1% (v/v) Trypsin, and 0.02% (v/v) EDTA was irreplaceable for rapid isolation of adipose-derived stromal cells.

The above content sets forth many specific details in order to fully understand the present invention, however, the present invention may be implemented in many forms different from what is described; one skilled in the art may modify and vary the examples without departing from the spirit and scope of the present invention, therefore, the examples should not be construed as the limitation of the claims.

## Claims

1. A composition for isolating adipose-derived stromal cells, which comprises a Type I collagenase of 0.5-8% (v/v); a Trypsin of 0.1-0.6% (v/v); and a metal ion chelating agent of 0.01-0.2% (v/v).

2. The composition according to claim 1, wherein the metal ion chelating agent is selected from ethylenediaminetetraacetic acid (EDTA) or sodium salts thereof, ethylene glycol-bisaminoethyl ether tetraacetic acid (EGTA) or sodium salts thereof, diethylene triamine pentaacetic acid (DTPA) or sodium salts thereof, polyphosphates, organic phosphates, phosphates, polyacrylates, organic phosphates, sodium gluconate, or mixtures thereof.

3. The composition according to claim 2, wherein the metal ion chelating agent is EDTA.

4. A method for isolating adipose-derived stromal cells, which comprises the steps of:
(a) obtaining an adipose tissue;
(b) adding the composition according to claim 1, homogenizing and allowing for reaction to obtain a digested tissue mixture, wherein the composition comprises a Type I collagenase of 0.5-8% (v/v); a Trypsin of 0.1-0.6% (v/v); and a metal ion chelating agent of 0.01-0.2% (v/v);
(c) centrifuging the digested tissue mixture of step (b), removing impurities to obtain a first filtrate containing the adipose-derived stromal cells;
(d) adding a hypotonic solution to the first filtrate of step (c) to obtain a second filtrate of hemocyte-free adipose-derived stromal cells; and
(e) neutralizing the second filtrate of step (d) and centrifuging.

5. The method according to claim 4, wherein the metal ion chelating agent is selected from ethylenediaminetetraacetic acid (EDTA) or sodium salts thereof, ethylene glycol-bisaminoethyl ether tetraacetic acid (EGTA) or sodium salts thereof, diethylene triamine pentaacetic acid (DTPA) or sodium salts thereof, polyphosphates, organic phosphates, phosphates, polyacrylates, organic phosphates, sodium gluconate, or mixtures thereof.

6. The method according to claim 5, wherein the metal ion chelating agent is EDTA.

7. The method according to claim 4, which is further **characterized in that** the total reaction time for digesting an adipose tissue is one hour or less to obtain characteristics of the adipose-derived stromal cells.

8. The method according to claim 4, which has an effect of isolating at least one million adipose-derived stromal cells from 5 gram of adipose tissue.

9. The method according to claim 4, wherein the adipose-derived stromal cells are capable of differentiating into adipose cells, hematopoietic cells, vascular endothelial cells, osteoblasts, chondroblasts, nerve cells or epithelial cells.
